Europäisches Patentamt

European Patent Office

Office européen des brevets

(11) Publication number: 0 350 087
A1

# EUROPEAN PATENT APPLICATION

(21) Application number: 89201577.7

(22) Date of filing: 15.06.89

(51) Int. Cl.⁴: A61F 6/14

(30) Priority: 27.06.88 NL 8801623

(43) Date of publication of application:
10.01.90 Bulletin 90/02

(84) Designated Contracting States:
AT BE CH DE ES FR GB IT LI LU NL SE

(71) Applicant: FUNDATECH S.A.
7, Place de la Fusterie Case Postale 492
CH-1211 Geneva - 3(CH)

(72) Inventor: Spits, Marc
1, Rue Princesse Florestine
MC-98000 Monaco(MC)

(74) Representative: Kupecz, Arpad et al
Octrooibureau Los en Stigter B.V. Postbox
20052
NL-1000 HB Amsterdam(NL)

(54) Intra-uterine contraceptive and method for fabricating it.

(57) The invention relates to an intra-uterine contraceptive (1), which is provided with an elongated shaft (4), which at one of its ends carries at least two resilient freely supported arms (2), which laterally extend at both sides of the shaft (4) and wherein the shaft (4) is provided with a contraceptive coating. Said contraceptive (1) is characterized in that the other free end of the shaft (4) is designed as a stop means (6) for holding the contraceptive coating.

Advantageously the stop means (6) is a contra hook, whereas the contaceptive coating is a suitable metal wire spiral (5) or metal tube and preferably a copper wire spiral (5) or copper tube.

Usually the contraceptive coating covers the shaft (4) over its entire length.

Finally the present invention relates to a method for fabricating the intra-uterine contraceptive (1) of the invention.

fig.1

Xerox Copy Centre

## Intra-uterine contraceptive and method for fabricating it

The present invention relates to an intra-uterine contraceptive, which is provided with an elongated shaft, which at one of its ends carries at least two resilient freely supported arms, which laterally extend at both sides of the shaft and wherein the shaft is provided with a contraceptive coating, and to a method for fabricating such intra-uterine contraceptive.

Such intra-uterine contraceptive, usually called IUD, is known from Dutch patent specification 173707.

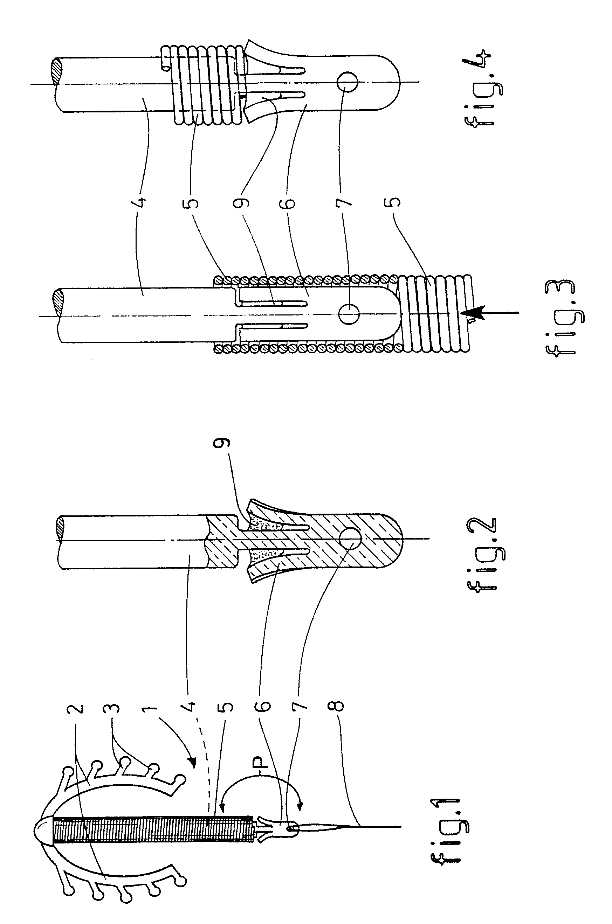

From fig. 1 of the just mentioned patent specification it appears that the shaft at the upper side at a certain distance from the end is provided with an eye. The copper wire which herein is used as the contraceptive coating was first threaded through the eye by hand, whereafter the copper wire by hand or mechanically was wound around the shaft.

It will be clear that fabricating this known IUD is cumbersome, since herein many manual operations, like manually threading the copper wire in the eye, securing the wire, cutting the wire later on, have to be done.

Another disadvantage which may be mentioned is that the spiral obtained therewith shows irregularities, which may lead to an IUD of different activity.

Another disadvantage of the known IUD is the fact that the upper end of the shaft is not completely covered with copper wire, whereby there is no active material at the fundus of the uterus. All this results in that the contraceptive effect leaves a desire for improvement.

The invention has the object of providing an intra-uterine contraceptive, wherein the disadvantage of the known intra-uterine contraceptive have been removed in an efficient manner.

For this purpose the invention comprises an intra-uterine contraceptive which is provided with an elongated shaft, which at one of its ends carries at least two resilient freely supported arms, which laterally extend at both sides of the shaft and wherein the shaft is provided with a contraceptive coating, characterized in that the other free end of the shaft is designed as a stop means for holding the contraceptive coating.

The lower end of the shaft of the intra-uterine contraceptive is designed as a stop means, so that the contraceptive coating is locked, whereby this coating is prevented from leaving the shaft and coming to rest in the uterus.

The securing eye of, for example, the copper wire at the upper end of the shaft of the intra-uterine contraceptive is superfluous according to the invention, since the lower end of the shaft is designed as a stop means.

Preferably, the stop means is embodied as a contra hook.

As a contraceptive coating usually a suitable metal wire spiral or metal tube is considered, while a copper wire spiral or copper tube is preferred, because of the particularly favourable effect of this metal for preventing conception.

It has appeared particularly favourable when the contraceptive coating covers the shaft over its entire length, whereby active material is present at the fundus of the uterus, which leads to an increased contraceptive effect.

Furthermore, the invention relates to a method for fabricating an intra-uterine contraceptive. The method of the invention is characterized in that the first the contraceptive without the contraceptive coating is fabricated by injecting moulding form a suitable plastic, whereafter the contraceptive coating in the form of a suitable metal wire spiral or metal tube of suitable length is disposed on the shaft via the stop means, whereby the spiral or tube is locked by the stop means.

According to the present method the intra-uterine contraceptive of the invention can be fabricated in a simple and reproducible manner.

In respect of production technique the present method has considerable advantages in relation to the known methods. The suitable metal wire spiral, copper spiral for example, of the right length is separately fabricated in a manner known per se. Herein spirals of different composition, different length and different material can be fabricated dependent on the envisaged use, as well of the person who will use the intra-uterine contraceptive.

It will be clear that by separately fabricating the spirals great regularity can be assured, which also is favourable.

Since according to the method of the invention ready-made spirals can be used, very simple production changes can take place dependent on the kinds of wire, wire diameter, pitch etc.

The invention will now be further explained by way of the drawing, wherein

Fig. 1 illustrates a favourable embodiment of the intra-uterine contraceptive of the invention,

Fig. 2 illustrates an enlarged part of the stop means without spiral,

Fig. 3 shows the stop means, while the spiral is slid, and

Fig. 4 shows the position of the stop means after the spiral is slid on the shaft.

In fig. 1 the intra-uterine contraceptive 1 of the invention is provided with an elongated shaft 4, which at one of its ends carries two resilient freely supported arms 2, which laterally extend on both sides of the shaft 4. The contraceptive itself is held in the uterus cavity by means of projections 3, which are disposed at the outer side of the arms 2. In this embodiment the entire length of the shaft is covered by a copper spiral 5, which spiral is locked by the stop means 6, so that the spiral after having been disposed in the uterus cannot leave the shaft. The stop means 6 herein is embodied like a contra-hook, which provides for the locking of the spiral.

For easily removing the intra-uterine contraceptive from the uterus a pull thread 8 is fixed to the lower end of the shaft by eye 7.

It will be clear that instead of a spiral also a metal tube of suitable length and composition may be used. Furthermore metal rings may be used.

Fig. 2 is an enlarged view of the shaft part of fig. 1 indicated by P, which clearly shows that the stop means 6 is integrally formed with the shaft 4. Between the central part of the shaft 4 and the contra-hook there is a flexible web 9. This web assures a good stopping effect of the stop means 6.

Fig. 3 clearly shows that during sliding of the spiral 5 the stop means 6 is pressed inwardly, so that the spiral 5 easily can be slid, whereas fig. 4 shows the situation after the spiral 5 is brought to its definitive position. After passing of the stop means 6 the stop means unfolds itself and prevents the spiral from leaving the shaft 4. It is clearly visible that the web 9 prevents the contra-hooks of the stop means 6 from expanding too far.

It will be clear that the invention is not limited to the above-mentioned embodiment.

## Claims

1. Intra-uterine contraceptive, which is provided with an elongated shaft, which at one of its ends carries at least two resilient freely supported arms, which laterally extend at both sides of the shaft and wherein the shaft is provided with a contraceptive coating, characterzed in that the other free end of the shaft is designed as a stop means for holding the contraceptive coating.

2. Intra-uterine contraceptive of claim 1, characterized in that the stop means is a contra hook.

3. Intra-uterine contraceptive of claim 1 or 2, characterized in that the contraceptive coating is a suitable metal wire spiral or metal tube.

4. Intra-uterine contraceptive of claim 3, characterized in that the contraceptive coating is a copper wire spiral or copper tube.

5. Intra-uterine contaceptive of claims 1 - 4, characterized in that the contraceptive coating covers the shaft over its entire length.

6. Method for fabricating the intra-uterine contraceptive of claims 1 - 5, characterised in that first the contraceptive without the contraceptive coating is fabricated by injecting moulding from a suitable plastic, whereafter the contraceptive coating in the from of a suitable metal wire spiral or metal tube of suitable length is disposed on the shaft via the stop means, whereby the spiral or tube is locked by the stop means.

fig.1

fig.2

fig.3

fig.4

EP 0 350 087 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 147 274 (BIOMASYS)<br>* Abstract; claim 25; page 13, line 21 - page 4, line 27 *<br>--- | 1,3-6 | A 61 F 6/14 |
| A | GB-A-2 024 020 (MEDHOLDING)<br>* Figures 4,5; page 2, lines 32-54 *<br>--- | 1 | |
| A | FR-A-2 263 738 (BENNE)<br>* Figures 1,5-7 *<br>--- | 1,2 | |
| A | US-A-4 553 536 (ENRICO)<br>* Abstract; figure 1; column 1, lines 54-56 *<br>----- | | |

TECHNICAL FIELDS SEARCHED (Int. Cl.5)

A 61 F

The present search report has been drawn up for all claims

| Place of search | Date of completion of the search | Examiner |
|---|---|---|
| THE HAGUE | 26-09-1989 | STEENBAKKER J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)